# EUROPEAN PATENT APPLICATION

(11) **EP 4 173 631 A1**
(43) Date of publication of application: **03.05.2023**
(21) Application number: 21832923.3
(22) Date of filing: 30.06.2021
(51) Int. Cl.: A61K 35/51, A61K 9/19, A61K 38/19, A61K 38/20, A61P 25/00, A61P 43/00

(54) **CRANIAL NEUROPATHY THERAPEUTIC AGENT CONTAINING CULTURE SUPERNATANT FOR UMBILICAL CORD BLOOD MONOCYTIC CELLS**

(30) Priority: 30.06.2020 JP 2020113322
(71) Applicant: National University Corporation Kochi University, Kochi-shi, Kochi 780-8520 (JP)
(72) Inventor: SAGARA, Yusuke, Kochi-shi, Kochi 780-8520 (JP)
(74) Representative: advotec.
(86) International application number: PCT/JP2021/024854
(87) International publication number: WO 2022/004823

(57) **Abstract**

Provided is a cranial neuropathy therapeutic agent that contains, as an active ingredient, a culture supernatant for umbilical cord blood monocytic cells.

## Description

### FIELD

The present invention relates to a therapeutic agent for brain disorder comprising a culture supernatant of umbilical cord blood mononuclear cells.

### BACKGROUND

Neonatal brain disorders during the perinatal period occur at a frequency of 1 or 2 per 1000 child births, causing lifelong cerebral palsy and creating a major burden not only on the individual but also on the family that raises them.

The perinatal brain injury that is causative of cerebral palsy includes hypoxic-ischemic encephalopathy, cerebral hemorrhage and periventricular leukomalacia, the major pathologies being intracellular Ca increase, active oxygen increase caused by mitochondrial function insufficiency, and inflammation of hypercytokinemia accompanying macrophage activation. Cerebral hemorrhage is a common complication of premature infancy, and its symptoms are the most severe with poor life prognosis.

Hypothermia treatment is considered to be an effective method, but has an efficacy rate of only about one in 8 to 9 infants. No method of treatment other than hypothermia has yet been established.

Clinical research is progressing in the area of direct administration of autologous bone marrow-derived mesenchymal cells into cerebral palsy patients, with some efficacy being reported (NPL 1). Administration of umbilical cord-derived cells has also been reported, but no significant improvements have been observed in certain motor functions (NPL 2). Significant amelioration in motor function impairment has also been reported by administration of umbilical cord blood-derived cells to neonatal cerebral hemorrhage model mice (PTL 1).

### [CITATION LIST]

### [NON PATENT LITERATURE]

[NPL 1] Cytotherapy 2013 Dec; 15(12):1549-62
[NPL 2] Cytotherapy 2015 17(2): 224-231

### [PATENT LITERATURE]

[PTL 1] International Patent Publication No. WO2017/204231

### SUMMARY

### [TECHNICAL PROBLEM]

In cerebral hemorrhagic or ischemic cranial nerve disorder, it is necessary not only to cure the hemorrhage and ischemia cause but also to protect and promote regeneration of the neurons that are damaged as a result. It has been considered to administer umbilical cord-derived cells for protection and regeneration of such neurons, but due to the different characteristics of cells obtained by methods of harvesting from umbilical cords, it has been unclear what effects are to be obtained by different types of cells derived from umbilical cords. Intrasubarachnoid administration can also be conducted by lumbar puncture for cranial nerve disorder, but this method of administration is risky and therefore therapeutic agents that exhibit effects by easier routes of administration are desired.

### [SOLUTION TO PROBLEM]

The present inventors have conducted much diligent research with the aim of solving the problems described above, and have completed this invention upon finding that a culture supernatant of umbilical cord blood mononuclear cells promotes homing of brain neural stem cells to brain disorder sites and differentiation and maturation of brain neural stem cells, and thereby regenerates cranial nerves and is effective for cranial nerve disorders.

Specifically, the present invention relates to the following:
(1) A cranial nerve disorder therapeutic agent comprising a culture supernatant of umbilical cord blood mononuclear cells as an active ingredient.
(2) The cranial nerve disorder therapeutic agent according to (1), wherein the culture supernatant comprises cytokines and chemokines.
(3) The cranial nerve disorder therapeutic agent according to (1) or (2), comprising IL-6, CXCL1, CXCL7, CXCL8 and CCL2.
(4) The cranial nerve disorder therapeutic agent according to any one of (1) to (3), wherein the culture supernatant is obtained by culturing umbilical cord blood mononuclear cells for 1 to 3 days.
(5) The cranial nerve disorder therapeutic agent according to any one of (1) to (4), wherein the density of umbilical cord blood mononuclear cells is 5 × 10⁴ to 5 × 10⁶/mL at the start of culturing.
(6) The cranial nerve disorder therapeutic agent according to any one of (1) to (5), wherein the cranial nerve disorder is non-hereditary cranial nerve damage.
(7) The cranial nerve disorder therapeutic agent according to any one of (1) to (6), wherein the cranial nerve disorder is dyskinesia (levodopa-induced dyskinesia, chronic or tardive dyskinesia, or orofacial dyskinesia), restless leg syndrome (drug-induced or idiopathic), drug-induced dystonia, chorea (Huntington's disease, toxin-induced chorea, Sydenham chorea, chorea of pregnancy, Wilson's disease, drug-induced chorea or metabolic or endocrine chorea), facial spasm (such as mobile, phonetic, simple, complex or Tourette's syndrome), dystonia (such as acute, systemic, localized, segmental, sexual, neutral, psychogenic or acute dystonic reaction), Sodemytopic Parkinson's disease, stereotypic movement disorder (such as autism or heredity or childhood-related movement disorder), obsessive-compulsive disorder, narcolepsy (such as cataplexy), transmissible spongiform encephalopathy (such as Creutzfeldt-Jakob disease or kuru), dementia (such as Alzheimer's, Lewy body dementia, vascular dementia, Pick's disease or alcoholic dementia), neuroacanthocytosis, seizure or convulsion, athetosis (such as Huntington's disease, respiratory arrest, neonatal jaundice- or stroke-related) or cerebral palsy.
(8) A method for producing a cranial nerve disorder therapeutic agent according to any one of (1) to (7), the method including (a) a step of culturing umbilical cord blood mononuclear cells, and (b) a step of recovering the culture supernatant of umbilical cord blood mononuclear cells.
(9) A pharmaceutical composition comprising a cranial nerve disorder therapeutic agent according to any one of (1) to (7).
(10) The pharmaceutical composition according to (9), which is a lyophilized preparation.
(11) A cranial nerve regeneration promoter comprising a culture supernatant of umbilical cord blood mononuclear cells as an active ingredient.
(12) The cranial nerve regeneration promoter according to (11), wherein the cranial nerve regeneration promoter is a cranial neural progenitor cell proliferating promoter, a cranial neural progenitor cell homing promoter, a cranial neural progenitor cell differentiation/maturation promoter, or a combination thereof.
(13) The cranial nerve regeneration promoter according to (11) or (12), wherein the culture supernatant comprises cytokines and chemokines.
(14) The cranial nerve regeneration promoter according to any one of (11) to (13), comprising IL-6, CXCL1, CXCL7, CXCL8 and CCL2.
(15) The cranial nerve regeneration promoter according to any one of (11) to (14), wherein the culture supernatant is obtained by culturing umbilical cord blood mononuclear cells for 1 to 3 days.
(16) The cranial nerve regeneration promoter according to any one of (11) to (15), wherein the density of umbilical cord blood mononuclear cells is 5 × 10⁴ to 5 × 10⁶/mL at the start of culturing.
(17) The cranial nerve regeneration promoter according to any one of (11) to (16), wherein the cranial nerve regeneration occurs with non-hereditary cranial nerve damage.
(18) The cranial nerve regeneration promoter according to any one of (11) to (17), wherein the cranial nerve regeneration occurs with dyskinesia (levodopa-induced dyskinesia, chronic or tardive dyskinesia, or orofacial dyskinesia), restless leg syndrome (drug-induced or idiopathic), drug-induced dystonia, chorea (Huntington's disease, toxin-induced chorea, Sydenham chorea, chorea of pregnancy, Wilson's disease, drug-induced chorea or metabolic or endocrine chorea), facial spasm (such as mobile, phonetic, simple, complex or Tourette's syndrome), dystonia (such as acute, systemic, localized, segmental, sexual, neutral, psychogenic or acute dystonic reaction), Sodemytopic Parkinson's disease, stereotypic movement disorder (such as autism or heredity or childhood-related movement disorder), obsessive-compulsive disorder, narcolepsy (such as cataplexy), transmissible spongiform encephalopathy (such as Creutzfeldt-Jakob disease or kuru), dementia (such as Alzheimer's, Lewy body dementia, vascular dementia, Pick's disease or alcoholic dementia), neuroacanthocytosis, seizure or convulsion, athetosis (such as Huntington's disease, respiratory arrest, neonatal jaundice- or stroke-related), or cerebral palsy.
(19) A method for producing a cranial nerve regeneration promoter according to any one of (11) to (18), the method including (a) a step of culturing umbilical cord blood mononuclear cells, and (b) a step of recovering the culture supernatant of umbilical cord blood mononuclear cells.
(20) A pharmaceutical composition comprising a cranial nerve regeneration promoter according to any one of (11) to (18).
(21) The pharmaceutical composition according to (20), which is a lyophilized preparation.

### [ADVANTAGEOUS EFFECTS OF INVENTION]

According to the invention it is possible to provide a therapeutic agent for cranial nerve disorder, comprising a culture supernatant of umbilical cord blood mononuclear cells. The formulation can be combined with numerous donor cell culture supernatants to produce a homogeneous cranial nerve disorder therapeutic agent, and can be more easily produced, stored and administered compared to cell-containing formulations.

### BRIEF DESCRIPTION OF DRAWINGS

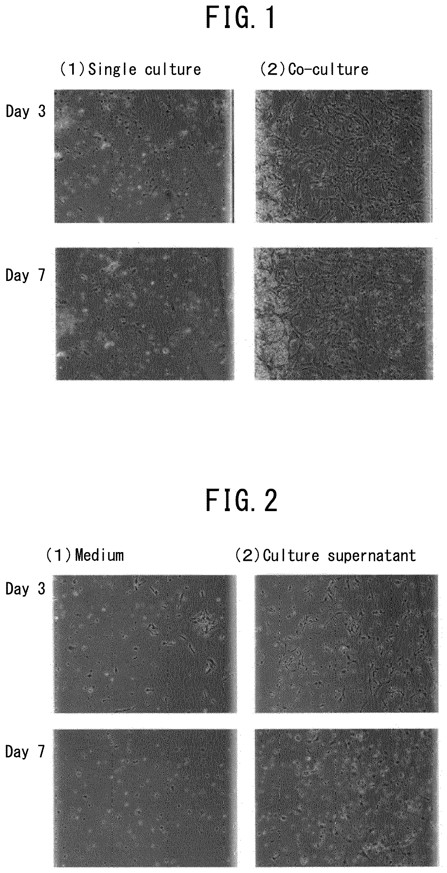

Fig. 1 shows nerve regeneration by co-culturing of umbilical cord blood mononuclear cells and neural progenitor cells. With co-culturing (2), proliferation, survival, movement (migration) and differentiation/maturation of neural progenitor cells were accelerated on the 3rd day and 7th day of culturing, compared to single culture (1).
Fig. 2 shows nerve regeneration (*in vitro*) by an umbilical cord blood mononuclear cell culture supernatant. With addition of culture supernatant (2), proliferation, survival, movement (migration) and differentiation/maturation of neural progenitor cells were accelerated on the 3rd day and 7th day of culturing, compared to addition of medium alone (1).

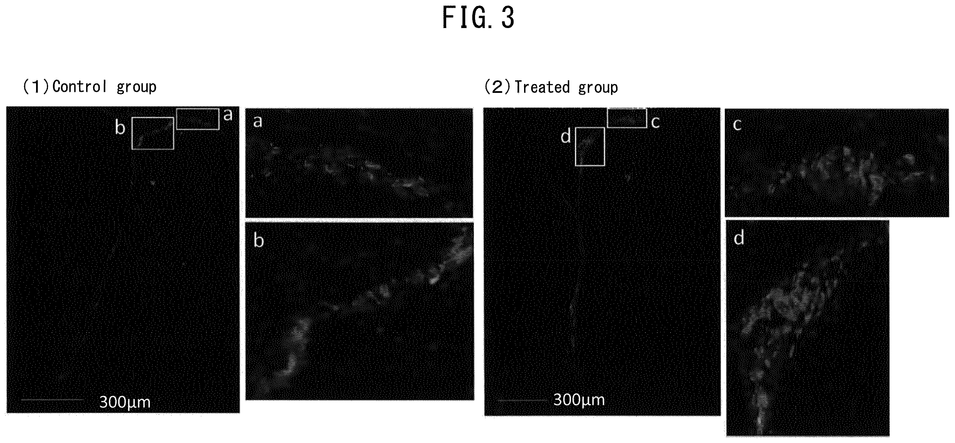

Fig. 3 shows nerve regeneration (subventricular zone) by an umbilical cord blood mononuclear cell culture supernatant. Administration of culture supernatant to hypoxic pediatric paralysis model mice (2) accelerated proliferation of neural progenitor cells in the subventricular zone compared to administration of medium alone (1) ((a), (b) vs (c), (d)).

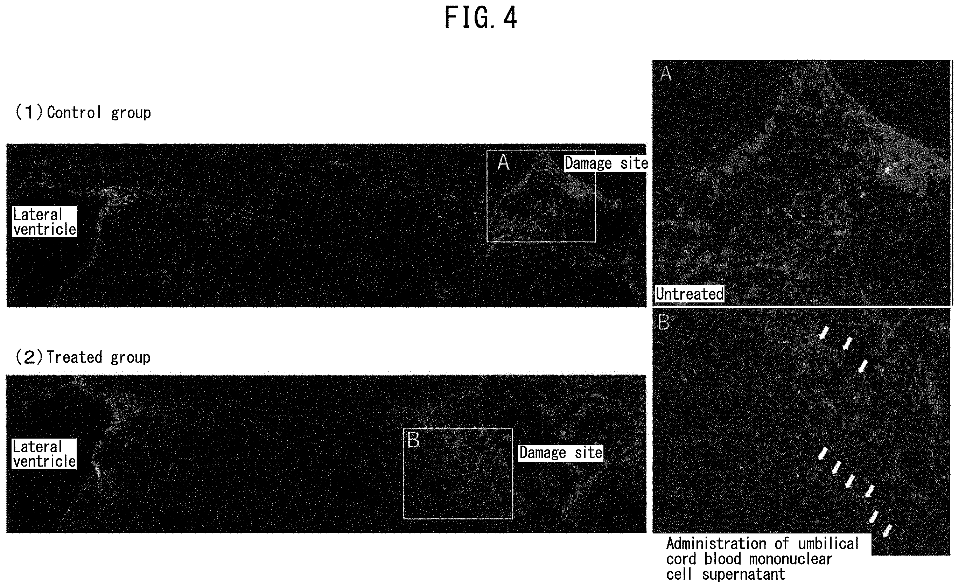

Fig. 4 shows nerve regeneration (brain damage site) by an umbilical cord blood mononuclear cell culture supernatant. Administration of culture supernatant to hypoxic pediatric paralysis model mice (2) accelerated homing of neural progenitor cells around the brain damage site and differentiation/maturation of neural progenitor cells to neurons around the brain damage site, compared to administration of medium alone (1) (arrow (B)).

### DESCRIPTION OF EMBODIMENTS

The invention will now be described in greater detail by concrete embodiments. However, the invention is not restricted to the described embodiments and may be carried out in any form within a range that is not outside of the gist of the invention.

All of the references including patent publications, patent applications and non-patent publications cited throughout the present disclosure are invoked in their entirety and are incorporated herein for all purposes.

For the purpose of the present disclosure, the word "to" when used between numerical values indicates a range that is above and including the lower value and is below and including the upper value.

### Umbilical cord blood mononuclear cells

The term "umbilical cord" as used herein means the white tubular tissue that connects the fetus and placenta, but does not include the placenta. The umbilical cord of the invention is not particularly restricted so long as it is harvested from a mammal, and it may be a primate mammalian umbilical cord, for example. It is more preferably a human umbilical cord.

The term "umbilical cord blood" as used herein means fetal blood present in the umbilical cord, while "human umbilical cord blood" means blood that can be obtained from a human umbilical cord. Human umbilical cord blood can be prepared from umbilical cord collected after appropriate removal of the placenta from postpartum tissue which includes the placenta and umbilical cord, following either vaginal delivery or delivery by caesarean section. Human umbilical cord blood can also be obtained from a cord blood bank such as Hyogo Cord Blood Bank.

The term "umbilical cord blood mononuclear cells" as used herein means cells that are mononuclear and present among the cells in umbilical cord blood. Umbilical cord blood mononuclear cells can be separated from umbilical cord blood, by a method such as gravity centrifugation method (using Lympholye, Ficoll-Paque, Percoll or Lymphoprep, for example), a method using a cell sorter, or an antibody method such as immunomagnetic separation or immunogravity centrifugation. A dialysis membrane may also be used to separate the mononuclear cells from the umbilical cord blood. It is also possible to use a whole blood monocyte concentration Purecell Select System^{®} (PALL), a blood cell removing purifier (Cellsorba E^{®}, Asahi Kasei Corp.) or a platelet preparation leukocyte removal filter (SEPACELL PL^{®}, PLX-5B-SCD, Asahi Kasei Corp.), without being limitative. The umbilical cord blood mononuclear cells differ from different stem cell types that are derived from umbilical cord blood in that they do not require multigenerational subculturing or detailed cell fractionation and are thus easy to prepare.

### 2. Preparation of culture supernatant

The "culturing" of the umbilical cord blood mononuclear cells may be carried out by a common method such as the following, for example. The umbilical cord blood mononuclear cells are added to basal medium in a culture vessel also containing sodium, potassium, calcium, magnesium, phosphorus, chlorine, amino acids, vitamins, hormones, cytokines, antibiotics, fatty acids, sugars or other chemical components, or biological components such as serum, as necessary for the purpose, and cultured in a CO₂ incubator with adjustment to a culturing temperature of 30°C to 40°C and preferably 37°C, and a carbon dioxide gas concentration of 0.03% to 40%, preferably 5 to 10% and even more preferably 5%. The oxygen concentration may be the same concentration as air, or it may be a lower concentration (1 to 20%). For example, the oxygen concentration may be 1%, 2%, 3%, 4%, 5%, 6%, 7%, 8%, 9%, 10%, 11%, 12%, 13%, 14%, 15%, 16%, 17%, 18%, 19% or 20%. The culturing period may be from a short period of several hours up to 1 day, 1.5 days, 2 days, 3 days, 5 days or 7 days.

The medium (culture solution) used may be any one that does not inhibit proliferation, maintenance and survival of the umbilical cord blood mononuclear cells, or their secretion of proteins. The basal medium used may be Dulbecco's Modified Eagle Medium (DMEM), or Iscove's Modified Dulbecco's Medium (IMDM), Ham F12 medium (HamF12) or RPMI 1640 medium. Two or more basal media may also be used in combination. An example of mixed medium is an equivolume mixture of IMDM and HamF12 medium (for example, IMDM/HamF12). Examples of components to be added to the medium include serum (such as fetal bovine serum, human serum, horse serum or sheep serum), serum substitute (such as Knockout serum replacement), conditioned medium, bovine serum albumin, human serum albumin, antibiotics, vitamins, deep sea water or minerals.

For the purpose of the present disclosure, the medium for the umbilical cord blood mononuclear cells to be used for preparation of a cranial nerve disorder therapeutic agent preferably contains no serum. Using serum-free medium can lower the risk of infection by prions and the like and prevent contamination by heteroproteins such as bovine serum albumin, thereby increasing the safety of the cranial nerve disorder therapeutic agent. For example, culturing umbilical cord blood mononuclear cells in medium without serum (serum-free medium) allows culture supernatant to be prepared that is free of serum components.

The medium used to prepare the cranial nerve disorder therapeutic agent of the disclosure may include deep sea water. The term "deep sea water" generally refers to seawater at a location greater than a depth of 200 meters, which around the region of Greenland is the origin of vertically sinking sea currents on a global scale known as "plumes" which are produced by difference in salt concentration, and consists of seawater that has been moving over the Earth for many centuries without ever contacting the atmosphere. Deep sea water is virtually shielded from sunlight rays and is in a low temperature high-pressure state, free of bacteria and rich in inorganic nutrient salts (minerals). By including deep sea water it is possible to adjust the amount of secretory proteins in culture supernatant and to prepare a more highly active cranial nerve disorder therapeutic agent than when deep sea water is not included. The concentration (v/v) of deep sea water in the medium may be, for example, 1%, 2%, 3%, 4%, 5%, 6%, 7%, 8%, 9%, 10%, 15%, 20%, 25%, 30%, 40%, 50%, 60%, 70% or 80% or greater, or it may be 90%, 80%, 70%, 60%, 50%, 45%, 40%, 35%, 30%, 25%, 20%, 15% or 10% or lower, such as in the range of 1 to 85%, 5 to 85%, 10% to 85%, 15% to 85%, 20% to 85%, 25% to 85%, 30% to 85%, 40% to 85%, 50% to 85%, 60% to 85%, 70% to 85%, 80% to 85%, 1 to 75%, 5 to 75%, 10% to 75%, 15% to 75%, 20% to 75%, 25% to 75%, 30% to 75%, 40% to 75%, 50% to 75%, 60% to 75%, 70% to 75%, 1 to 45%, 5 to 45%, 10% to 45%, 15% to 45%, 20% to 45%, 25% to 45%, 30% to 45%, 40% to 45%, 1 to 10% or 5 to 10%, or it may be 73% or 81%.

The culturing time for obtaining the culture supernatant of umbilical cord blood mononuclear cells may be, for example, 5 hours to 7 days, 1 day to 6 days, 1 day to 5 days, 1 day to 4 days, 1 day to 3 days or 1 day to 2 days, or it may be 0.5 day, 1 day, 2 days, 3 days, 4 days, 5 days, 6 days or 7 days. The culturing temperature may be 36°C to 38°C, such as 37°C, and the CO₂ concentration may be 4 to 6%, such as 5%, for example. The culturing may be three-dimensional culturing under non-adhesive conditions, for example, or suspension culturing (such as dispersion culturing or aggregated suspension culturing).

The density of umbilical cord blood mononuclear cells at the start of culturing, to obtain a culture supernatant of umbilical cord blood mononuclear cells, may be 1 × 10⁴ to 1 × 10⁷/mL, 5 × 10⁴ to 5 × 10⁶/mL, 1 × 10⁵ to 5 × 10⁶/mL, 2 × 10⁵ to 5 × 10⁶/mL, 5 × 10⁵ to 5 × 10⁶/mL or 1 × 10⁶ to 5 × 10⁶/mL, such as 2 × 10⁵/mL, 3 × 10⁵/mL, 4 × 10⁵/mL, 5 × 10⁵/mL, 6 × 10⁵/mL, 7 × 10⁵/mL, 8 × 10⁵/mL, 9 × 10⁵/mL, 1 × 10⁶/mL, 2 × 10⁶/mL, 3 × 10⁶/mL, 4 × 10⁶/mL, 5 × 10⁶/mL, 6 × 10⁶/mL, 7 × 10⁶/mL, 8 × 10⁶/mL, 7 × 10⁶/mL, 8 × 10⁶/mL, 9 × 10⁶/mL or 1 × 10⁷/mL.

After culturing, the cell components may be separated and removed to obtain the culture supernatant of umbilical cord blood mononuclear cells. According to this disclosure, "culture supernatant" refers not only to the supernatant after separation and removal of the cell components from the culture solution, but also to culture supernatant that has been subjected to various types of treatment (such as centrifugation, concentration, solvent exchanged, dialysis, freezing, drying, freeze-drying, dilution, desalting and storage). Details regarding treatment methods for the culture supernatant are described below. Culture supernatant according to the disclosure does not contain the cell components. The culture supernatant of the disclosure therefore does not contain the umbilical cord blood mononuclear cells used for culturing, and is not a composition for cellular medicine.

The umbilical cord blood mononuclear cells of the disclosure secrete cytokines, chemokines and growth factors into the medium. Proteins present in the culture supernatant include Interleukin(IL)-6, C-X-C Motif Chemokine Ligand (CXCL)1, CXCL7, CXCL8 (IL-8) and C-C Motif Chemokine Ligand (CCL)2 (MCP-1), which are important for cranial nerve regeneration.

For the purpose of the disclosure, the culture supernatant of umbilical cord blood mononuclear cells may include one or more of the following proteins:
cytokines such as the TNF Superfamily Member 14 (LIGHT), Interleukin(IL)-1α, IL-1β, IL-2, IL-3, IL-4, IL-5, IL-7, IL-10, IL-12p40/70, IL-13, IL-15, IL-16, Interferon (IFN)-y, Tumor Necrosis Factor (TNF)-α and TNF-β;
chemokines such as C-X-C Motif Chemokine Ligand (CXCL)1/2/3 (GROα/β/γ), CXCL6, CXCL9 (MIG), CXCL10 (IP-10), CXCL12 (SDF-1), CXCL13, C-C Motif Chemokine Ligand (CCL)1, CCL4, CCL5, CCL7, CCL8, CCL11, CCL13, CCL15, CCL17, CCL18, CCL20 (LARC), CCL22, CCL23, CCL24, CCL26 and C-X3-C Motif Chemokine Ligand(CX3CL)1 (FKN);
growth factor/related factors such as Angiogenin, Brain Derived Neurotrophic Factor (BDNF), Epidermal Growth Factor (EGF), Fibroblast Growth Factor (FGF)-4, FGF-6, FGF-7, FGF-9, Fms Related Receptor Tyrosine Kinase-3 Ligand (FLT-3L), Glial Cell Derived Neurotrophic Factor (GDNF), Granulocyte-Macrophage Colony-Stimulating Factor (GM-CSF), Granulocyte Colony-Stimulating Factor (G-CSF), Hepatocyte Growth Factor (HGF), Insulin Like Growth Factor (IGF)-1, Insulin Like Growth Factor Binding Protein (IGFBP)-2, IGFBP-1, IGFBP-4, IGFBP-3, Platelet Derived Growth Factor (PDGF)-BB, Placental Growth Factor (PLGF), Transforming Growth Factor (TGF)-β1, TGF-β2, TGF-β3, Thrombopoietin (TPO), Stem Cell Factor (SCF), Macrophage Colony Stimulating Factor (M-CSF), Neurotrophin (NT)-3, NT-4 and Vascular Endothelial Growth Factor (VEGF)-A; and
other mediators such as Leptin, Leukemia Inhibitory Factor (LIF), Macrophage Migration Inhibitory Factor (MIF), Osteopontin (OPN), Osteoprotegerin (OPG), Oncostatin M (OSM), Tissue Inhibitor of Metalloproteinases (TIMP)-1 and TIMP-2.

Factors that may be included in the culture supernatant of umbilical cord blood mononuclear cells of the disclosure are important for nerve regeneration for the following reasons. The culture supernatant of the disclosure includes all or some of these factors, that are suitable for brain or central nerve regeneration (Boruczkowski et al., Int. J. Mol. Sci. 2019 20, 2433; Watson et al., Neuroscience Letters 2020 715, 134533; Wang et al., Stem Cell Research & Therapy 2017 8, 26; Baba et al., PLOS ONE 2019 14(9)e0221111; Wang et al., Acta Medica Okayama 2012 66(6) 429).
a) Proliferation and survival of neural progenitor cells: BDNF, CCL5, CXCL1, CXCL7, CXCL8, CX3CL1, EGF, FGF-9, G-CSF, IGF-1, IL-6, OPN.
b) Differentiation and maturation of neural progenitor cells: BDNF, CCL11, CXCL1, CXCL7, CXCL9, CXCL12, CX3CL1, FGF-4, FGF-9, GDNF, GM-CSF, IL-1β, LIF, OPN, SCF, TGF-β3.
c) Migration of neural progenitor cells: CCL2, CCL11, CCL20, CXCL1, CXCL7, CXCL8, CXCL12, EGF, IGF-1.
d) Neurotrophy: GDNF, NGF, NT-4/5.
e) Activation of astrocytes: BDNF, IFN-y, IL-1α, IL-1β.
f) Activation of microglial cells: CCL17, CXCL10, Leptin.
g) Regeneration of oligodendrocytes: CXCL1, CXCL7, CXCL8, CXCL12, CX3CL1, EGF, M-CSF, NT-4, IGFBP-4.
h) Vascularization: angiogenin, CCL2, CXCL5, CXCL6, CXCL7, CXCL8, Groα/β/γ, HGF, IGF-1, IL-6, VEGF, SCF.
i) Anti-inflammation: CCL4, IL-10, IL-13, TGF-β2.

According to the disclosure, the culture supernatant of umbilical cord blood mononuclear cells which is a mixture of cytokines and chemokines (and growth factors) can be used as one portion of the umbilical cord blood mononuclear cell culture supernatant, or as a mixture of cytokines and chemokines (and growth factors) isolated from the umbilical cord blood mononuclear cell culture supernatant. A mixture of cytokines and chemokines (and growth factors) isolated from the umbilical cord blood mononuclear cell culture supernatant may have some of the cytokines and chemokines (and growth factors) replaced by one or more corresponding known gene-recombinant cytokines and chemokines (and growth factors), or corresponding gene-recombinant cytokines and chemokines (and growth factors) may be added.

### 3. Cranial nerve disorder therapeutic agent

By comprising a culture supernatant of umbilical cord blood mononuclear cells, the cranial nerve disorder therapeutic agent of the disclosure exhibits an effect of regenerating nerve tissue in cranial nerve disorder. The level of the effect is comparable in full or in part to the conventional effect of grafting umbilical cord blood stem cells. Surprisingly, the cranial nerve disorder therapeutic agent of the disclosure exhibits a general nerve regeneration effect across a wide range of cranial nerve tissues, by promoting proliferation of neural progenitor cells in the subventricular zone, homing to sites of cranial nerve disorder, and differentiation/maturation of neurons at sites of cranial nerve damage. The cranial nerve disorder therapeutic agent of the disclosure therefore is not limited to any specific cranial nerve disorder, and it can be used as a cranial nerve regeneration promoter for a wide range of cranial nerve disorders, to treat and alleviate the symptoms of cranial nerve disorders. The cranial nerve disorder therapeutic agent of the invention can effectively inhibit progression of cranial nerve disorder by administration before cranial nerve disorder, even in unknown stages of disease that may lead to such cranial nerve disorder. By using the cranial nerve disorder therapeutic agent of the disclosure in a habitual manner it is possible to treat a variety of cranial nerve disorders before onset and thus prevent, delay or alleviate their onset. The term "treat" as used herein includes not only treatment for complete curing of symptoms or anomalies associated with cranial nerve disorder, but also alleviation of symptoms or anomalies that do not result in complete curing, or treatment that delays them compared to lack of treatment, as well as prevention, delay or alleviation of onset.

Since the cranial nerve disorder therapeutic agent of the disclosure promotes nerve regeneration, it can be used for a wide range of types of cranial nerve disorders. Non-hereditary cranial nerve disorder is an example of cranial nerve disorder. Without being limitative, the cranial nerve disorder therapeutic agent of the disclosure can be applied for dyskinesia (levodopa-induced dyskinesia, chronic or tardive dyskinesia, or orofacial dyskinesia), restless leg syndrome (drug-induced or idiopathic), drug-induced dystonia, chorea (Huntington's disease, toxin-induced chorea, Sydenham chorea, chorea of pregnancy, Wilson's disease, drug-induced chorea or metabolic or endocrine chorea), facial spasm (such as mobile, phonetic, simple, complex or Tourette's syndrome), dystonia (such as acute, systemic, localized, segmental, sexual, neutral, psychogenic or acute dystonic reaction), Sodemytopic Parkinson's disease, stereotypic movement disorder (such as autism or heredity or childhood-related movement disorder), obsessive-compulsive disorder, narcolepsy (such as cataplexy), transmissible spongiform encephalopathy (such as Creutzfeldt-Jakob disease or kuru), dementia (such as Alzheimer's, Lewy body dementia, vascular dementia, Pick's disease or alcoholic dementia), neuroacanthocytosis, seizure or convulsion, athetosis (such as Huntington's disease, respiratory arrest, neonatal jaundice- or stroke-related), or cerebral palsy.

The target of administration of the cranial nerve disorder therapeutic agent will typically be a human patient with damage to target tissue, but the agent may also be applied to a mammal other than a human (including pet animals, livestock and experimental animals, and specifically mice, rats, guinea pigs, hamsters, monkeys, cows, pigs, goats, sheep, dogs or cats).

There are no particular restrictions on the site where the cranial nerve disorder has occurred. The diseased site may be all or part of the brain, the brain including both the forebrain and brain stem, and the forebrain including the cerebrum and diencephalon, and the brain stem including the midbrain and hindbrain. The cerebrum includes the olfactory brain, amygdala, striatum, hippocampus and cerebral neocortex, and the diencephalon includes the epithalamus, thalamus, hypothalamus, thalamus abdomen, pituitary, pineal body and third cerebral ventricle. The midbrain includes the mesencephalon, cerebral peduncle, preoptic area and cerebral aqueduct, and the hindbrain includes the pons, cerebellum and medulla oblongata. A site of cranial nerve disorder may be any of these sites.

The treatment method using the cranial nerve disorder therapeutic agent of the disclosure includes intranasal administration of the umbilical cord blood mononuclear cell culture supernatant for repair of the site of brain damage. This treatment method allows low-invasive and effective restoration of regions suffering from injury due to cranial nerve disorder such as cerebral palsy.

### 4. Production method

The disclosure provides a method for producing a cranial nerve disorder therapeutic agent which includes:
(a) a step of culturing umbilical cord blood mononuclear cells, and (b) a step of recovering the culture supernatant of the umbilical cord blood mononuclear cells. The production method may further include a step of carrying out one or more processes selected from among centrifugation, concentration, solvent exchange, dialysis, freezing, drying, freeze-drying, dilution and desalting of the collected culture supernatant. Including these steps will further facilitate handling, storage and transport of the cranial nerve disorder therapeutic agent. The production method may also include a step of adding additional components to the collected culture supernatant. Addition of other components can alter the physical properties and improve the properties of the composition for treatment of cranial nerve disorder. The production method described above may further include a step of preparing somatic umbilical cord blood mononuclear cells from umbilical cord blood. The conditions described for the cranial nerve disorder therapeutic agent of the disclosure all apply for each of these steps and additional components. When the method includes both a step of carrying out one or more processes selected from among centrifugation, concentration, solvent exchange, dialysis, freezing, drying, freeze-drying, dilution and desalting of the collected culture supernatant, and a step of adding additional components to the collected culture supernatant, the steps may be carried out in any order, or when possible they may be carried out simultaneously in parallel.

In step (b), the culture supernatant of umbilical cord blood mononuclear cells is collected. The culture solution may be collected by drawing with a dropper or pipette, for example. The collected culture supernatant is used as an active ingredient of the cranial nerve disorder therapeutic agent of the disclosure, either directly or after being treated by one or more treatments. The treatment may be, for example, centrifugation, concentration, solvent exchange, dialysis, formulation, freezing, drying, freeze-drying, dilution, desalting and storage (such as 4°C or -80°C). The culture supernatant of umbilical cord blood mononuclear cells functions as expected even without complex advanced purification. The cranial nerve disorder therapeutic agent of the disclosure can therefore be prepared by a simple procedure. Eliminating the need for complex purification is advantageous because it avoids the reduction in activity that occurs with purification.

The umbilical cord blood mononuclear cell culture supernatant of the disclosure may also be derived from umbilical cord blood mononuclear cells obtained from multiple donors. Specifically, umbilical cord blood mononuclear cells taken from multiple donors may be combined and cultured, and the resulting culture supernatant collected, or alternatively the culture supernatants of umbilical cord blood mononuclear cells taken from individual donors may be combined, and then subjected to one or more treatments (centrifugation, concentration, solvent exchange, dialysis, formulation, freezing, drying, freeze-drying, dilution, desalting or storage), subsequently combining the culture supernatants of umbilical cord blood mononuclear cells derived from the individual donors. However, because the umbilical cord blood mononuclear cell culture supernatant of the disclosure can potentially provoke excessive mixed lymphocyte reaction, it is preferred not to culture umbilical cord blood mononuclear cells from multiple donors in admixture. Culture supernatant of umbilical cord blood mononuclear cells from multiple donors is advantageous in that it can be produced as a homogeneous cranial nerve disorder therapeutic agent.

### <Method of concentrating umbilical cord blood mononuclear cell culture supernatant>

The composition for treatment of cranial nerve disorder of the disclosure may be a formulated preparation. The method of concentrating umbilical cord blood mononuclear cell culture supernatant for formulation may be a method commonly used for concentration of culture supernatants. The following two methods are examples of concentration methods.

### 1. Spin column concentration

The culture supernatant is concentrated using Amicon Ultra Centrifugal Filter Units-10K (Millipore). The specific procedure is as follows.
(i) The culture supernatant (maximum 15 mL) is loaded into Amicon Ultra Centrifugal Filter Units-10K and centrifuged at ×4000 g for about 60 minutes for concentration to 200 µL.
(ii) The culture supernatant and an equivolume of sterilized PBS are loaded into the tube, and centrifugation is repeated at ×4000 g for about 60 minutes, replacing the base solution with the PBS.
(iii) A 200 µL portion of the obtained solution is collected in a micro test tube as the concentrated umbilical cord blood mononuclear cell culture supernatant.

### 2. Ethanol precipitation concentration

The culture supernatant is concentrated by ethanol precipitation. The specific procedure is as follows.
(i) A 45 mL portion of 100% ethanol is added to and mixed with 5 mL of culture supernatant, and the mixture is allowed to stand at -20°C for 60 minutes.
(ii) Centrifugation is carried out at 4°C × 15,000 g for 15 minutes.
(iii) The supernatant is removed, 10 mL of 90% ethanol is added and the mixture is thoroughly stirred.
(iv) Centrifugation is carried out at 4°C × 15,000 g for 5 minutes.
(v) The supernatant is removed, and the obtained pellet is dissolved in 500 µL of sterilized water and collected in a micro test tube as the concentrated umbilical cord blood mononuclear cell culture supernatant.

### <Method of freeze-drying umbilical cord blood mononuclear cell culture supernatant>

The umbilical cord blood mononuclear cell culture supernatant for the cranial nerve disorder therapeutic agent of the disclosure may also be freeze-dried. This will help provide more satisfactory storage stability. The method of freeze-drying the umbilical cord blood mononuclear cell culture supernatant may be a method commonly used for freeze-drying of culture supernatants. The following method is an example of a freeze-drying method. The freeze-dried culture supernatant may be used as a powder formulation or it may be reconstituted for use in a suitable solvent such as water.
(i) Umbilical cord blood mononuclear cell culture supernatant or concentrated umbilical cord blood mononuclear cell culture supernatant obtained as described above is frozen at -80°C for a period of 2 to 12 hours.
(ii) After freezing, the sample tube cover is opened and the tube is set in a freezing dryer.
(iii) Freeze-drying is carried out for 1 to 2 days.
(iv) The obtained sample is used as a freeze-dried umbilical cord blood mononuclear cell culture supernatant (storable at -80°C).

### 5. Pharmaceutical composition

The cranial nerve disorder therapeutic agent of the disclosure may also include other components for a pharmaceutical composition, for the purpose of supporting the expected curative effect in light of the condition of the subject to which is to be applied. Examples of additional components include the following.

### (i) Bioabsorbable materials

Hyaluronic acid, collagen and fibrinogen (such as BOLHEAL^{™}) may be used as organic bioabsorbable materials.

### (ii) Gelling materials

Gelling materials are preferably highly biocompatible materials, such as hyaluronic acid, collagen or fibrin paste. Various types of hyaluronic acid or collagen may be selected for use, but they are preferably selected as suited for the purpose of use (the target tissue) of the cranial nerve disorder therapeutic agent of the disclosure. The collagen used is preferably soluble (acid-soluble collagen, alkali-soluble collagen or enzyme-soluble collagen).

### (iii) Other components

Other pharmaceutically acceptable components (for example, carriers, excipients, disintegrators, buffering agents, emulsifying agents, suspending agents, soothing agents, stabilizers, preservatives, antiseptic agents, physiological saline and the like) may also be added. Excipients include lactose, starch, sorbitol, D-mannitol and saccharose. Disintegrators that may be used include starches, carboxymethyl cellulose and calcium carbonate. Buffering agents that may be used include phosphates, citrates and acetates. Emulsifying agents that may be used include gum arabic, sodium alginate and tragacanth. Suspending agents that may be used include glycerin monostearate, aluminum monostearate, methyl cellulose, carboxymethyl cellulose, hydroxymethyl cellulose and sodium lauryl sulfate. Soothing agents that may be used include benzyl alcohol, chlorobutanol and sorbitol. Stabilizers that may be used include propylene glycol and ascorbic acid. Preservatives that may be used include phenol, benzalkonium chloride, benzyl alcohol, chlorobutanol and methylparaben. Antiseptic agents that may be used include benzalkonium chloride, paraoxybenzoic acid and chlorobutanol. Antibiotics, pH adjustors, growth factors (such as epidermal growth factor (EGF), nerve growth factor (NGF) and brain-derived neurotrophic factor (BDNF)) may also be added.

The final form of the pharmaceutical composition of the disclosure is not particularly restricted. Examples of dosage forms include liquid forms (liquids and gels) and solid forms (including lyophilized preparations such as powders, fine grains and granules). The pharmaceutical composition of the disclosure may also be in a form suited for inhalation, in which case it may be in a liquid form that is dispersible as a mist by a nebulizer or diffuser. The site of cranial nerve disorder is the brain, and in light of the presence of the blood brain barrier, the composition for treatment of cranial nerve disorder according to the disclosure is preferably in a form that can be administered intranasally, intracerebroventricularly or intrathecally. For example, it may be administered intranasally in the form of a spray or powder. From the viewpoint of prior preparation and storage, the culture supernatant of umbilical cord blood mononuclear cells is more advantageous than using umbilical cord blood mononuclear cells or umbilical cord blood stem cells, and may therefore be considered especially suitable for treatment of acute or subacute cranial nerve disorder. The culture supernatant of umbilical cord blood mononuclear cells is also highly useful from the viewpoint of overcoming immunological rejection as well, since it does not contain cell components.

Depending on the embodiment, however, the cranial nerve disorder therapeutic agent of the disclosure may still include umbilical cord blood mononuclear cells, umbilical cord blood stem cells, ES cells, iPS cells or mesenchymal stem cells in addition to the culture supernatant of umbilical cord blood mononuclear cells, or it may be used in tandem with such stem cells. Using stem cells in addition may further improve the therapeutic effect.

The present disclosure also provides a method of inhibiting onset of cranial nerve disorder in a subject prior to onset of the cranial nerve disorder, the method comprising administration of the cranial nerve disorder therapeutic agent of the disclosure at a dose effective for preventing onset of the cranial nerve disorder, before onset of the cranial nerve disorder. The subject may be a human, or a mammal other than a human (such as a pet animal, farm animal or experimental animal). Specifically, it may be mouse, rat, guinea pig, hamster, monkey, cow, pig, goat, sheep, dog or cat). The subject may also be one assessed to be at risk for onset of cranial nerve disorder. Such risk can be assessed by gene diagnosis or genealogical analysis. For example, it has been statistically shown that the presence of specific alleles or SNPs in specific genes are correlated with greater likelihood of specific diseases.

The dose of the cranial nerve disorder therapeutic agent of the disclosure is not limited so long as the expected therapeutic effect is maintained, and it may be 0.1 mL/kg/day to 100 mL/kg/day, 1 mL/kg/day to 100 mL/kg/day or 5 mL/kg/day to 100 mL/kg/day, in terms of the amount of untreated culture supernatant, or 0.1 mg/kg/day to 1000 mg/kg/day or 1 mg/kg/day to 100 mg/kg/day, in terms of the protein level of the untreated culture supernatant. The method of administration is also not particularly restricted. The cranial nerve disorder therapeutic agent is preferably administered by parenteral administration, for example, with either systemic or local administration as the method of parenteral administration. Examples of methods of administering the cranial nerve disorder therapeutic agent are not particularly restricted so long as the expected therapeutic effect is maintained, and it may be intravenous administration, intraarterial administration, intraportal administration, intradermal administration, subcutaneous administration, intramuscular administration, intraperitoneal administration, transpulmonary administration (transpulmonary absorption), intracerebroventricular administration, intrathecal administration or nasal administration, for example. Intranasal administration is preferred because it has low invasiveness. Intracerebroventricular administration, intrathecal administration and intranasal administration can ensure passage through the blood brain barrier and are therefore particularly effective for treatment of cranial nerve disorder. The dosing schedule may be once or several times per day, or once every two days, or once every three days. The gender, age, body weight and pathology of the subject may be considered when creating the dosing schedule.

### 6. Cranial nerve regeneration promoter

The umbilical cord blood mononuclear cell culture supernatant of the disclosure is a cranial nerve regeneration promoter. The term "cranial nerve regeneration" refers to at least one of many phenomena during the course of development, including nerve repair or nerve development by increase, differentiation and maturation of neural progenitor cells in the brain. Nerve regeneration therefore preferably includes the phenomenon of complete or partial recovery of original nerve function. A publicly known method may be used to confirm that nerve regeneration has been efficiently achieved. For example, a patient or livestock animal having nerve injury and having been administered a nerve regeneration promoter may be compared with a patient or livestock animal having nerve injury and not having been administered a nerve regeneration promoter, and it may be judged that efficient nerve regeneration has been achieved if the patient or livestock animal that has been administered the nerve regeneration promoter has greater recovery in the function of the damaged nerve. Recovery of nerve function can be evaluated based on reaction to stimulation or recovery of motor function or cognitive function, as demonstrated in the Examples below. Recovery of motor function can be examined by a publicly known method, such as a Rotarod test (Oh et al., Exp. Mol. Med. 2018 50(4), 22; Kossatz et al., Front. Pharmacol. 2018 9, 376), a Y-maze test (Miedel et al., J. Vis. Exp. 2017(123); Sarnyai et al., Proc. Natl. Acad. Sci. USA 2000 97(26), 14731) or a hanging wire test (Aartsma-Rus et al., J. Vis. Exp. 2014(85)).

Nerve regeneration may be by cells derived from defective nerves that are originally from the site to be treated (endogenous cells), or cells that have been grafted together with the nerve regeneration promoter (exogenous cells). Such cells include neurons, neural progenitor cells, embryonic stem cells, induced pluripotent stem cells, mesenchymal stem cells, vascular endothelial cells, vascular endothelial precursor cells and hematopoietic stem cells.

The umbilical cord blood mononuclear cell culture supernatant of the disclosure, as a cranial nerve regeneration promoter, may be a cranial neural progenitor cell proliferating promoter. The term "cranial neural progenitor cell proliferating promoter" means an agent that promotes proliferation of neural progenitor cells in the brain. A publicly known method may be employed to confirm that proliferation of cranial neural progenitor cells has been effectively achieved. For example, proliferation of neural progenitor cells in the brain or derived from the brain by addition of the cranial neural progenitor cell differentiation/maturation promoter can be evaluated by observation, as in the Examples described below. Neural progenitor cells harvested from a living body, or cells differentiated from stem cells such as iPS cells, or a cell line (such as A172, B65, C6, KS-1, N2A, PC12, SH-SY5Y, SKN-BE2, T98G, U251, U87 or YH-13) may be used.

The umbilical cord blood mononuclear cell culture supernatant of the disclosure, as a cranial nerve regeneration promoter, may also be a cranial neural progenitor cell homing promoter. The term "cranial neural progenitor cell homing promoter" means an agent that promotes migration of neural progenitor cells to a brain disorder site. A publicly known method may be employed to confirm that homing of cranial neural progenitor cells has been effectively achieved. For example, a patient or livestock animal promoter having nerve injury and having been administered a cranial neural progenitor cell homing promoter may be compared with a patient or livestock animal having nerve injury and not having been administered a cranial neural progenitor cell homing promoter, and it may be judged that efficient homing of cranial neural progenitor cells has been achieved if the patient or livestock animal that has been administered the cranial neural progenitor cell homing promoter is observed to have more migration of cranial neural progenitor cells to the brain disorder site from the hippocampal granule subcellular zone or the subventricular zone. Administration in combination with cells such as neural progenitor cells allows assessment to be made by accumulation of the administered cells in the brain disorder site as well. Homing of cranial neural progenitor cells can be evaluated using a cranial neural progenitor cell marker (such as DCX (doublecortin), SOX2 (SRY (sex determining region Y)-box 2) or PAX6 (paired box 6)), as described in the Examples below.

The umbilical cord blood mononuclear cell culture supernatant of the disclosure, as a cranial nerve regeneration promoter, may also be a cranial neural progenitor cell differentiation/maturation promoter. The term "cranial neural progenitor cell differentiation/maturation promoter" means an agent that promotes axon elongation of neural progenitor cells in the brain and their differentiation and maturation into neurons.

A publicly known method may be employed to confirm that differentiation and maturation of cranial neural progenitor cells has been effectively achieved. For example, elongation of axons of neural progenitor cells in the brain or derived from the brain by addition of the cranial neural progenitor cell differentiation/maturation promoter can be evaluated by observation or by measurement of the lengths of the extended axons, as in the Examples described below. Neural progenitor cells harvested from a living body, or cells differentiated from stem cells such as iPS cells, or a cell line (such as A172, B65, C6, KS-1, N2A, PC12, SH-SY5Y, SKN-BE2, T98G, U251, U87 or YH-13) may be used.

The cranial nerve regeneration promoter of the disclosure includes cytokines and chemokines. Cytokines and chemokines include Interleukin(IL)-6, C-X-C Motif Chemokine Ligand (CXCL)1, CXCL7, CXCL8 (IL-8) and C-C Motif Chemokine Ligand (CCL)2 (MCP-1), which are important for cranial nerve regeneration.

The cranial nerve regeneration promoter of the disclosure may also include one or more of the following proteins:
cytokines such as the TNF Superfamily Member 14 (LIGHT), Interleukin(IL)-1α, IL-1β, IL-2, IL-3, IL-4, IL-5, IL-7, IL-10, IL-12p40/70, IL-13, IL-15, IL-16, Interferon (IFN)-y, Tumor Necrosis Factor (TNF)-α and TNF-β;
chemokines such as C-X-C Motif Chemokine Ligand (CXCL)1/2/3 (GROα/β/γ), CXCL6, CXCL9 (MIG), CXCL10 (IP-10), CXCL12 (SDF-1), CXCL13, C-C Motif Chemokine Ligand (CCL)1, CCL4, CCL5, CCL7, CCL8, CCL11, CCL13, CCL15, CCL17, CCL18, CCL20 (LARC), CCL22, CCL23, CCL24, CCL26 and C-X3-C Motif Chemokine Ligand (CX3CL)1 (FKN);
growth factor/related factors such as Angiogenin, Brain Derived Neurotrophic Factor (BDNF), Epidermal Growth Factor (EGF), Fibroblast Growth Factor (FGF)-4, FGF-6, FGF-7, FGF-9, Fms Related Receptor Tyrosine Kinase-3 Ligand (FLT-3L), Glial Cell Derived Neurotrophic Factor (GDNF), Granulocyte-Macrophage Colony-Stimulating Factor (GM-CSF), Granulocyte Colony-Stimulating Factor (G-CSF), Hepatocyte Growth Factor (HGF), Insulin Like Growth Factor (IGF)-1, Insulin Like Growth Factor Binding Protein (IGFBP)-2, IGFBP-1, IGFBP-4, IGFBP-3, Platelet Derived Growth Factor (PDGF)-BB, Placental Growth Factor (PLGF), Transforming Growth Factor (TGF)-β1, TGF-β2, TGF-β3, Thrombopoietin (TPO), Stem Cell Factor (SCF), Macrophage Colony Stimulating Factor (M-CSF), Neurotrophin (NT)-3, NT-4 and Vascular Endothelial Growth Factor (VEGF)-A; and
other mediators such as Leptin, Leukemia Inhibitory Factor (LIF), Macrophage Migration Inhibitory Factor (MIF), Osteopontin (OPN), Osteoprotegerin (OPG), Oncostatin M (OSM) and Tissue Inhibitor of Metalloproteinases (TIMP)-1 and TIMP-2.

The medium used to prepare the cranial nerve regeneration promoter of the disclosure may include deep sea water. The term "deep sea water" generally refers to seawater at a location greater than a depth of 200 meters, which around the region of Greenland is the origin of vertically sinking sea currents on a global scale known as "plumes" which are produced by difference in salt concentration, and consists of seawater that has been moving over the Earth for many centuries without ever contacting the atmosphere. Deep sea water is virtually shielded from sunlight rays and is in a low temperature high-pressure state, free of bacteria and rich in inorganic nutrient salts (minerals). By including deep sea water it is possible to adjust the amount of secretory proteins in culture supernatant and to prepare a more highly active cranial nerve disorder therapeutic agent than when deep sea water is not included. The concentration (v/v) of deep sea water in the medium may be, for example, 1%, 2%, 3%, 4%, 5%, 6%, 7%, 8%, 9%, 10%, 15%, 20%, 25%, 30%, 40%, 50%, 60%, 70% or 80% or greater, or it may be 90%, 80%, 70%, 60%, 50%, 45%, 40%, 35%, 30%, 25%, 20%, 15% or 10% or lower, such as in the range of 1 to 85%, 5 to 85%, 10% to 85%, 15% to 85%, 20% to 85%, 25% to 85%, 30% to 85%, 40% to 85%, 50% to 85%, 60% to 85%, 70% to 85%, 80% to 85%, 1 to 75%, 5 to 75%, 10% to 75%, 15% to 75%, 20% to 75%, 25% to 75%, 30% to 75%, 40% to 75%, 50% to 75%, 60% to 75%, 70% to 75%, 1 to 45%, 5 to 45%, 10% to 45%, 15% to 45%, 20% to 45%, 25% to 45%, 30% to 45%, 40% to 45%, 1 to 10% or 5 to 10%, or it may be 73% or 81%.

The culturing time for obtaining the culture supernatant of umbilical cord blood mononuclear cells as a cranial nerve regeneration promoter may be, for example, 5 hours to 7 days, 1 day to 6 days, 1 day to 5 days, 1 day to 4 days, 1 day to 3 days or 1 day to 2 days, or it may be 0.5 day, 1 day, 2 days, 3 days, 4 days, 5 days, 6 days or 7 days. The culturing temperature may be 36°C to 38°C, such as 37°C, and the CO₂ concentration may be 4 to 6%, such as 5%, for example. The culturing may be three-dimensional culturing under non-adhesive conditions, for example, or suspension culturing (such as dispersion culturing or aggregated suspension culturing).

The density of umbilical cord blood mononuclear cells as a cranial nerve regeneration promoter at the start of culturing, to obtain a culture supernatant of umbilical cord blood mononuclear cells, may be 1 × 10⁴ to 1 × 10⁷/mL, 5 × 10⁴ to 5 × 10⁶/mL, 1 × 10⁵ to 5 × 10⁶/mL, 2 × 10⁵ to 5 × 10⁶/mL, 5 × 10⁵ to 5 × 10⁶/mL or 1 × 10⁶ to 5 × 10⁶/mL, such as 2 × 10⁵/mL, 3 × 10⁵/mL, 4 × 10⁵/mL, 5 × 10⁵/mL, 6 × 10⁵/mL, 7 × 10⁵/mL, 8 × 10⁵/mL, 9 × 10⁵/mL, 1 × 10⁶/mL, 2 × 10⁶/mL, 3 × 10⁶/mL, 4 × 10⁶/mL, 5 × 10⁶/mL, 6 × 10⁶/mL, 7 × 10⁶/mL, 8 × 10⁶/mL, 7 × 10⁶/mL, 8 × 10⁶/mL, 9 × 10⁶/mL or 1 × 10⁷/mL.

Since the cranial nerve regeneration promoter of the disclosure promotes nerve regeneration it can be used for a wide range of types of cranial nerve disorder. Non-hereditary cranial nerve disorder is an example of cranial nerve disorder. Without being limitative, the cranial nerve disorder therapeutic agent of the disclosure can be applied for dyskinesia (levodopa-induced dyskinesia, chronic or tardive dyskinesia, or orofacial dyskinesia), restless leg syndrome (drug-induced or idiopathic), drug-induced dystonia, chorea (Huntington's disease, toxin-induced chorea, Sydenham chorea, chorea of pregnancy, Wilson's disease, drug-induced chorea or metabolic or endocrine chorea), facial spasm (such as mobile, phonetic, simple, complex or Tourette's syndrome), dystonia (such as acute, systemic, localized, segmental, sexual, neutral, psychogenic or acute dystonic reaction), Sodemytopic Parkinson's disease, stereotypic movement disorder (such as autism or heredity or childhood-related movement disorder), obsessive-compulsive disorder, narcolepsy (such as cataplexy), transmissible spongiform encephalopathy (such as Creutzfeldt-Jakob disease or kuru), dementia (such as Alzheimer's, Lewy body dementia, vascular dementia, Pick's disease or alcoholic dementia), neuroacanthocytosis, seizure or convulsion, athetosis (such as Huntington's disease, respiratory arrest, neonatal jaundice- or stroke-related), or cerebral palsy.

The disclosure provides a method for producing a cranial nerve regeneration promoter which includes:
(a) a step of culturing umbilical cord blood mononuclear cells, and (b)
a step of recovering the culture supernatant of the umbilical cord blood mononuclear cells.
The production method may further include a step of carrying out one or more processes selected from among centrifugation, concentration, solvent exchange, dialysis, freezing, drying, freeze-drying, dilution and desalting of the collected culture supernatant. Including these steps will further facilitate handling, storage and transport of the cranial nerve regeneration promoter. The production method may also include a step of adding additional components to the collected culture supernatant. Addition of other components can alter the physical properties and improve the properties of the composition for promoting cranial nerve regeneration. The production method described above may further include a step of preparing somatic umbilical cord blood mononuclear cells from umbilical cord blood. The conditions described for the cranial nerve disorder therapeutic agent of the disclosure all apply for each of these steps and additional components. When the method includes both a step of carrying out one or more processes selected from among centrifugation, concentration, solvent exchange, dialysis, freezing, drying, freeze-drying, dilution and desalting of the collected culture supernatant, and a step of adding additional components to the collected culture supernatant, the steps may be carried out in any order, or when possible they may be carried out simultaneously in parallel.

The cranial nerve regeneration promoter of the disclosure may also include other components for a pharmaceutical composition, for the purpose of supporting the expected curative effect in light of the condition of the subject to which it is to be applied. Examples of additional components include the following.

### (i) Bioabsorbable materials

Hyaluronic acid, collagen and fibrinogen (such as BOLHEAL^{™}) may be used as organic bioabsorbable materials.

### (ii) Gelling materials

Gelling materials are preferably highly biocompatible materials, such as hyaluronic acid, collagen or fibrin paste. Various types of hyaluronic acid or collagen may be selected for use, but they are preferably selected as suited for the purpose of use (the target tissue) of the cranial nerve regeneration promoter of the disclosure. The collagen used is preferably soluble (acid-soluble collagen, alkali-soluble collagen or enzyme-soluble collagen).

### (iii) Other components

Other pharmaceutically acceptable components (for example, carriers, excipients, disintegrators, buffering agents, emulsifying agents, suspending agents, soothing agents, stabilizers, preservatives, antiseptic agents, physiological saline and the like) may also be added. Excipients include lactose, starch, sorbitol, D-mannitol and saccharose. Disintegrators that may be used include starches, carboxymethyl cellulose and calcium carbonate. Buffering agents that may be used include phosphates, citrates and acetates. Emulsifying agents that may be used include gum arabic, sodium alginate and tragacanth. Suspending agents that may be used include glycerin monostearate, aluminum monostearate, methyl cellulose, carboxymethyl cellulose, hydroxymethyl cellulose and sodium lauryl sulfate. Soothing agents that may be used include benzyl alcohol, chlorobutanol and sorbitol. Stabilizers that may be used include propylene glycol and ascorbic acid. Preservatives that may be used include phenol, benzalkonium chloride, benzyl alcohol, chlorobutanol and methylparaben. Antiseptic agents that may be used include benzalkonium chloride, paraoxybenzoic acid and chlorobutanol. Antibiotics, pH adjustors, growth factors (such as epidermal growth factor (EGF), nerve growth factor (NGF) and brain-derived neurotrophic factor (BDNF)) may also be added.

The final form of the pharmaceutical composition of the cranial nerve regeneration promoter of the disclosure is not particularly restricted. Examples of dosage forms include liquid forms (liquids and gels) and solid forms (including lyophilized preparations such as powders, fine grains and granules). The pharmaceutical composition of the disclosure may also be in a form suited for inhalation, in which case it may be in a liquid form that is dispersible as a mist by a nebulizer or diffuser. The site of cranial nerve disorder is the brain, and in light of the presence of the blood brain barrier, the composition for promoting cranial nerve regeneration according to the disclosure is preferably in a form that can be administered intranasally, intracerebroventricularly or intrathecally. For example, it may be administered intranasally in the form of a spray or powder. From the viewpoint of prior preparation and storage, the culture supernatant of umbilical cord blood mononuclear cells is more advantageous than using umbilical cord blood mononuclear cells or umbilical cord blood stem cells, for example, and may therefore be considered especially suitable for treatment of acute or subacute cranial nerve disorder. The culture supernatant of umbilical cord blood mononuclear cells is also highly useful from the viewpoint of overcoming immunological rejection as well, since it does not contain cell components.

### EXAMPLES

The present invention will now be explained in greater detail by examples. However, the invention is in no way limited by the Examples.

### Example 1: Preparation of umbilical cord blood mononuclear cells and culture supernatants

### 1) Preparation of human umbilical cord blood mononuclear cells

Umbilical cord blood mononuclear cells were prepared in the following manner. Specifically, 1 mL of heparin sodium injection (1,000 U/mL) was placed in a 50-mL polypropylene tube, and approximately 30 mL of human umbilical cord blood provided from Kochi University Medical School Hospital, Dept. of Obstetrics and Gynecology, with informed consent, was used. The harvested human umbilical cord blood was diluted 3 times with Hank's Balanced Salt Solution (HBSS). The diluted umbilical cord blood was carefully layered in a 50-mL polypropylene tube containing previously added 15 mL Lympholyte-H (Cedarlane), and centrifuged at 800 × g for 20 minutes. The separated mononuclear cell layer was collected, HBSS was added, the mixture was centrifuged at 300 × g for 10 minutes, and the cells were rinsed. After then adding 1 mL of the cell cryopreservation solution STEM-CELL BANKER (Takarabio) to the obtained mononuclear cells, the resulting suspension was cryopreserved in an ultra-low temperature freezer at -80°C. The cryopreserved cells were first thawed, and then either HBSS or 10% fetal bovine serum (FBS) was added, depending on the purpose of the experiment, and the cells were rinsed with the respective cell culture solution for use.

### 2) Culturing of human umbilical cord blood mononuclear cells

Umbilical cord blood mononuclear cells were suspended in DMEM medium or RPMI 1640 medium at a density of 2 × 10⁶ cells/mL, and cultured for 24 or 48 hours under conditions of 37°C, 5% CO₂, with or without addition of deep sea water. The deep sea water used was "Amami Water 1000" (Ako Kasei Co., Ltd.) taken offshore of Muroto City, Kochi prefecture. A NucleoCounter NC-100 (ChemoMetec Co.) was used to measure the cell viability, and while the results showed time-dependent reduction in survival rate, no difference was seen between addition and non-addition of the deep sea water. The medium was recovered and centrifuged at 300 G for 10 minutes to prepare a culture supernatant.

### Example 2: Nerve regeneration by co-culturing of umbilical cord blood mononuclear cells and neural progenitor cells

### 1) Preparation of neural progenitor cells

Neonatal NOD/SCID mice (Charles River, Japan: NOD.CB17-Prkdc ^{scid}/J) were euthanized 1 week after birth, and brain tissue was harvested. Surrounding tissue in the subventricular zone was cut out and dispersed using neuronal dispersion (Sumitomo Dainippon Pharma) and the obtained cells were cultured in NeuroCult Basal Complete Medium (Stemcell Technologies) with addition of 20 ng/mL epidermal growth factor (EGF), 10 ng/mL fibroblast growth factor (FGF) and 2 µL /mL heparin (Stemcell Technologies) at 37°C, 5% CO₂, forming a neural stem cell mass which was provided for the subsequent test.

### 2) Co-culturing

The human umbilical cord blood mononuclear cells prepared in Example 1 and the neural progenitor cells prepared in Example 2 were each suspended in NeuroCult Basal Complete Medium (Stemcell Technologies), and a µ-Slide Chemotaxis (Ibidi Co.) was used for co-culturing under conditions of 37°C, 5% CO₂, without contact between the cells. Proliferation and differentiation/maturation of the neural stem cells were evaluated on the 3rd and 7th days of culturing by microscope observation. As shown in Fig. 1, the umbilical cord blood mononuclear cells and co-cultured neural progenitor cells exhibited more active proliferation, with observation of greater movement (migration) of neural progenitor cells, compared to culturing alone, while greater differentiation and maturation to axon-extended neurons was also observed.

These results suggested that umbilical cord blood mononuclear cells in the prepared state (i.e. with all of the cell types including stem cells present without being isolated) secrete factors that promote proliferation, survival, movement (migration), differentiation and maturation of neural progenitor cells.

### Example 3: Nerve regeneration (in vitro) by umbilical cord blood mononuclear cell culture supernatant

### Effect on neural progenitor cells

The neural progenitor cells prepared in Example 2 were suspended in NeuroCult Basal complete medium (Stemcell Technologies) and seeded in a µ-Slide Chemotaxis (Ibidi Co.), and culture supernatant of the human culture supernatant of umbilical cord blood mononuclear cells obtained in Example 1 was added at 50% (v/v) prior to culturing under conditions of 37°C, 5% CO₂. As a control, the same medium used for preparation of the umbilical cord blood mononuclear cell culture supernatant was added in the same amount. Proliferation and differentiation/maturation of the neural progenitor cells were evaluated on the 3rd and 7th days of culturing by microscope observation. As shown in Fig. 2, compared to the control, the neural progenitor cells to which culture supernatant of umbilical cord blood mononuclear cells was added showed greater extension of axons on the 3rd day of culturing and greater proliferation or survival maintenance of the cells on the 3rd and 7th days of culturing, as well as greater movement (migration) of the neural progenitor cells.

These results suggested that umbilical cord blood mononuclear cells in the prepared state (i.e. with all of the cell types including stem cells present without being isolated) secrete factors that promote proliferation, survival, movement (migration), differentiation and maturation of neural progenitor cells, and that culture supernatant of umbilical cord blood mononuclear cells accelerates nerve regeneration.

### Factors in culture supernatants

The factors in the culture supernatant of umbilical cord blood mononuclear cells prepared in Example 1 were examined using an antibody array (Human Cytokine Array C5 by RayBiotech) (n = 6). As shown in Table 1, cytokines such as IL-1β and IL-6, chemokines such as CCL2, CCL5, CXCL1, CXCL5, CXCL7, CXCL8 and GROα/β/γ, growth factors such as BDNF and EGF, and Osteopontin (OPN) were present in the culture supernatants, suggesting that these factors promote nerve regeneration. When culture supernatants were prepared in medium with addition of deep sea water at 8, 41, 73 and 81%, the concentrations of some or all of these factors increased.

**[Table 1]**

| Factor | Relative concentration | Factor | Relative concentration | Factor | Relative concentration | Factor | Relative concentration |
|---|---|---|---|---|---|---|---|
| Angiogenin | 58 | CXCL5 | 331 | IGFBP-1 | 50 | LIGHT | 59 |
| BDNF | 75 | CXCL6 | 38 | IGFBP-2 | 110 | M-CSF | 106 |
| CCL1 | 4 | CXCL7 | 574 | IGFBP-3 | 98 | MIF | 95 |
| CCL2 | 1504 | CXCL8 | 2257 | IGFBP-4 | 66 | NT-3 | 247 |
| CCL4 | 362 | CXCL9 | 86 | IL-1a | 27 | NT-4 | 98 |
| CCL5 | 459 | CXCL10 | 161 | IL-1b | 155 | OPG | 79 |
| CCL7 | 53 | CXCL12 | 30 | IL-2 | 53 | OPN | 145 |
| CCL8 | 32 | CXCL13 | 44 | IL-3 | 44 | OSM | 68 |
| COL11 | 66 | EGF | 355 | IL-4 | 34 | PDGF-BB | 42 |
| COL13 | 53 | FGF-4 | 36 | IL-5 | 53 | PLGF | 75 |
| COL15 | 10 | FGF-6 | 80 | IL-6 | 2716 | SCF | 39 |
| CCL17 | 78 | FGF-7 | 38 | IL-7 | 83 | TGF-b1 | 41 |
| CCL18 | 87 | FGF-9 | 99 | IL-10 | 66 | TGF-b2 | 258 |
| CCL20 | 65 | FLT-3L | 26 | IL-12 | 19 | TGF-b3 | 76 |
| CCL22 | 75 | G-CS F | 41 | IL-13 | 19 | TIMP-1 | 251 |
| CCL23 | 57 | GDNF | 61 | IL-15 | 62 | TIMP-2 | 172 |
| CCL24 | 70 | GM-CSF | 37 | IL-16 | 64 | TNF-a | 113 |
| CCL26 | 54 | GRO a/b/g | 629 | INF-g | 44 | TNF-b | 86 |
| CX3CL1 | 34 | HGF | 43 | Leptin | 36 | TPO | 31 |
| CXCL1 | 209 | IGF-1 | 57 | LIF | 157 | VEGF-A | 57 |

### Example 4: Nerve regeneration (in vivo) by umbilical cord blood mononuclear cell culture supernatant

### 1) Creation of mouse hypoxic reperfusion pediatric cerebral palsy model

ICR mice (Crl: CD1, Charles River, Japan) were used to create hypoxic reperfusion pediatric cerebral palsy model mice based on the method of Rice-Vannucci et al. (Wang et al., J Matern Fetal Neonatal Med. 2015; 28(7):842-7). Mice (female and male) at 9 days after birth were anesthetized with 2% isoflurane, and the right carotid arteries were infarcted with a cerebral aneurysm clip (Mizuho Co.). Each individual was left for 120 minutes under 8% oxygen concentration, after which the cerebral artery clip was released and reperfusion of blood flow was initiated.

### Treatment with umbilical cord blood culture supernatant

Hypoxic pediatric paralysis model mice were intranasally administered 10 µL of the umbilical cord blood culture supernatant prepared in Example 1, every other day for a period of 2 weeks. The treated mice were used in a Rotarod test (MK-610A by Muromachi Kikai Co., Ltd.), measuring the time until falling from a rotor accelerated to 80 rpm in 300 seconds, and a tendency toward recovery was exhibited in the culture supernatant-administered group (ambulation times of 72, 90 and 112% before treatment, 1 week after treatment and 2 weeks after treatment, respectively, with respect to the ambulation time of healthy mice (n = 9)).

### Pathological evaluation

Treated hypoxic reperfusion pediatric cerebral palsy model mice (n = 3) were administered 100 mg/kg of bromodeoxyuridine (BrdU) to label proliferated cells. The brains of the mice were then extracted and fixed with 4% paraformaldehyde solution. A microtome (product of Leica) was used to produce 12 µm-thick frozen sections. Rabbit anti-mouse Doublecortin (Dcx) antibody (Abcam, 1:1000-fold dilution) and Alexa Fluor488-labeled goat anti-rabbit IgG antibody (Themo Fisher Scientific Waltham, 1:1000-fold dilution) were used for immunostaining of neural progenitor cells, mouse anti-mouse glial fibrillary acidic protein (GFAP) antibody (BD Biosciences, Cat. No. 556329, 1:50-fold dilution) and Alexa Flour 555-labeled goat anti-mouse IgG antibody (Cell Signaling Technology, 1:500-fold dilution) were used for immunostaining of glial cells, and anti-BrdU antibody (Abcam, 1:500-fold dilution) and Alexa Flour 555-labeled goat anti-rat IgG antibody (Cell Signaling Technology, 1:1000-fold dilution) were used for immunostaining of proliferating cells.

As shown in Fig. 3, Dcx/BrdU-co-positive neural progenitor cells were found in the subventricular zone region in the culture supernatant-administered group, with the neural progenitor cell count increasing to 2.1 times compared to the control group (group intranasally administered the same amount of medium used for preparation of the umbilical cord blood mononuclear cell culture supernatant) (treated group: 397 cells/slice, control group: 189 cells/slice). This indicated migration of the neural progenitor cells near to and accumulating (homing) at the brain disorder site with time.

As shown in Fig. 4, accumulation of astrocytes was observed around the brain damage sites of the hypoxic pediatric paralysis model mice, but in the culture supernatant-administered group, numerous proliferated neural progenitor cells with Dcx/BrdU-co-positivity were found, exhibiting a neuron-like cytomorphology around the astrocytes (treated group: 61 cells/slice, control group: 32 cells/slice).

The results of immunostaining of the brains of the hypoxic pediatric paralysis model mice suggested that the administered umbilical cord blood mononuclear cell culture supernatant promoted proliferation of neural progenitor cells in the subventricular zone, while also promoting homing to the brain disorder site and differentiation and maturation to neurons.

It was thus demonstrated that umbilical cord blood mononuclear cells in the prepared state (i.e. with all of the cell types including umbilical cord blood stem cells present without being isolated) secrete factors that promote proliferation, survival, migration/homing, differentiation and maturation of neural progenitor cells, and that culture supernatant of umbilical cord blood mononuclear cells can be used to regenerate nerves and to treat cranial nerve disorders such as cerebral palsy.

## Claims

1. A cranial nerve disorder therapeutic agent comprising a culture supernatant of umbilical cord blood mononuclear cells as an active ingredient.

2. The cranial nerve disorder therapeutic agent according to claim 1, wherein the culture supernatant comprises cytokines and chemokines.

3. The cranial nerve disorder therapeutic agent according to claim 1 or 2, comprising IL-6, CXCL1, CXCL7, CXCL8 and CCL2.

4. The cranial nerve disorder therapeutic agent according to any one of claims 1 to 3, wherein the culture supernatant is obtained by culturing umbilical cord blood mononuclear cells for 1 to 3 days.

5. The cranial nerve disorder therapeutic agent according to any one of claims 1 to 4, wherein the density of umbilical cord blood mononuclear cells is 5 × 10⁴ to 5 × 10⁶/mL at the start of culturing.

6. The cranial nerve disorder therapeutic agent according to any one of claims 1 to 5, wherein the cranial nerve disorder is non-hereditary cranial nerve disorder.

7. The cranial nerve disorder therapeutic agent according to any one of claims 1 to 6, wherein the cranial nerve disorder is dyskinesia (levodopa-induced dyskinesia, chronic or tardive dyskinesia, or orofacial dyskinesia), restless leg syndrome (drug-induced or idiopathic), drug-induced dystonia, chorea (Huntington's disease, toxin-induced chorea, Sydenham chorea, chorea of pregnancy, Wilson's disease, drug-induced chorea or metabolic or endocrine chorea), facial spasm (such as mobile, phonetic, simple, complex or Tourette's syndrome), dystonia (such as acute, systemic, localized, segmental, sexual, neutral, psychogenic or acute dystonic reaction), Sodemytopic Parkinson's disease, stereotypic movement disorder (such as autism or heredity or childhood-related movement disorder), obsessive-compulsive disorder, narcolepsy (such as cataplexy), transmissible spongiform encephalopathy (such as Creutzfeldt-Jakob disease or kuru), dementia (such as Alzheimer's, Lewy body dementia, vascular dementia, Pick's disease or alcoholic dementia), neuroacanthocytosis, seizure or convulsion, athetosis (such as Huntington's disease, respiratory arrest, neonatal jaundice- or stroke-related) or cerebral palsy.

8. A method for producing a cranial nerve disorder therapeutic agent according to any one of claims 1 to 7, the method including (a) a step of culturing umbilical cord blood mononuclear cells, and (b) a step of recovering the culture supernatant of umbilical cord blood mononuclear cells.

9. A pharmaceutical composition comprising a cranial nerve disorder therapeutic agent according to any one of claims 1 to 7.

10. The pharmaceutical composition according to claim 9, which is a lyophilized preparation.

11. A cranial nerve regeneration promoter comprising a culture supernatant of umbilical cord blood mononuclear cells as an active ingredient.

12. The cranial nerve regeneration promoter according to claim 11, wherein the cranial nerve regeneration promoter is a cranial neural progenitor cell proliferating promoter, a cranial neural progenitor cell homing promoter, a cranial neural progenitor cell differentiation/maturation promoter, or a combination thereof.

13. The cranial nerve regeneration promoter according to claim 11 or 12, wherein the culture supernatant comprises cytokines and chemokines.

14. The cranial nerve regeneration promoter according to any one of claims 11 to 13, comprising IL-6, CXCL1, CXCL7, CXCL8 and CCL2.

15. The cranial nerve regeneration promoter according to any one of claims 11 to 14, wherein the culture supernatant is obtained by culturing umbilical cord blood mononuclear cells for 1 to 3 days.

16. The cranial nerve regeneration promoter according to any one of claims 11 to 15, wherein the density of umbilical cord blood mononuclear cells is 5 × 10⁴ to 5 × 10⁶/mL at the start of culturing.

17. The cranial nerve regeneration promoter according to any one of claims 11 to 16, wherein the cranial nerve regeneration occurs with non-hereditary cranial nerve disorder.

18. The cranial nerve regeneration promoter according to any one of claims 11 to 17, wherein the cranial nerve regeneration occurs with dyskinesia (levodopa-induced dyskinesia, chronic or tardive dyskinesia, or orofacial dyskinesia), restless leg syndrome (drug-induced or idiopathic), drug-induced dystonia, chorea (Huntington's disease, toxin-induced chorea, Sydenham chorea, chorea of pregnancy, Wilson's disease, drug-induced chorea or metabolic or endocrine chorea), facial spasm (such as mobile, phonetic, simple, complex or Tourette's syndrome), dystonia (such as acute, systemic, localized, segmental, sexual, neutral, psychogenic or acute dystonic reaction), Sodemytopic Parkinson's disease, stereotypic movement disorder (such as autism or heredity or childhood-related movement disorder), obsessive-compulsive disorder, narcolepsy (such as cataplexy), transmissible spongiform encephalopathy (such as Creutzfeldt-Jakob disease or kuru), dementia (such as Alzheimer's, Lewy body dementia, vascular dementia, Pick's disease or alcoholic dementia), neuroacanthocytosis, seizure or convulsion, athetosis (such as Huntington's disease, respiratory arrest, neonatal jaundice- or stroke-related), or cerebral palsy.

19. A method for producing a cranial nerve regeneration promoter according to any one of claims 11 to 18, the method including (a) a step of culturing umbilical cord blood mononuclear cells, and (b) a step of recovering the culture supernatant of umbilical cord blood mononuclear cells.

20. A pharmaceutical composition comprising a cranial nerve regeneration promoter according to any one of claims 11 to 18.

21. The pharmaceutical composition according to claim 20, which is a lyophilized preparation.
